# EUROPÄISCHE PATENTSCHRIFT

(11) **EP 0 438 102 B1**
(45) Veröffentlichungstag und Bekanntmachung des Hinweises auf die Patenterteilung: **09.07.2003**
(21) Anmeldenummer: 91100376.2
(22) Anmeldetag: 14.01.1991
(51) Int. Cl.: C12N 15/13, C12P 21/08

(54) **Verfahren zur Antikörpergewinnung**
Process for the production of antibodies
Procédé de production d'anticorps

(30) Priorität: 15.01.1990 DE 4000939
(43) Veröffentlichungstag der Anmeldung: 24.07.1991
(73) Patentinhaber: apoGene GmbH, 86567 Hilgertshausen (DE)
(72) Erfinder: Weidle, Ulrich, Dr. rer. nat., W-8000 München 2 (DE); Brem, Gottfried Prof. Dr. med. vet., W-8893 Hilgertshausen (DE)
(74) Vertreter: Becker Kurig Straus

(56) Entgegenhaltungen:
- US-A- 4 870 009
- Proceedings of the National Academy of Sciences of the United States of America, Band 85, Nr. 9, Mai 1988 (Baltimore, USA), K.L. KNIGHT et al.: "Transgenic rabbits with lymphocytic leukemia induced by the c-myc oncogene fused with the immunoglobulin heavy chain enhancer", Seiten 3130-3134
- Zuchthygiene, 20. Jahrgang, 1985 (Berlin), G. BREM et al.: "Production of transgenic mice, rabbits and pigs by microinjection into pronuclei", Seiten 251-252
- Nature, Band 315, 20. Juni 1985 (New York), R.E. HAMMER et al.: "Production of transgenic rabbits, sheep and pigs by microinjection", Seiten 680-683

## Beschreibung

Zur Expression fremder DNA-Sequenzen in Tieren kann man die DNA in den männlichen Pronukleus von befruchteten Eizellen durch Mikroinjektion injizieren, die Eizellen dann in den Eileiter von entsprechenden Tieren implantieren und Nachkommen züchten, die das mikroinjizierte genetische Material exprimieren. Bei Mäusen wurden transgene Tiere auch durch Retrovirus-Infektion von Embryonen oder durch Transfer von genetisch manipulierten Stamm-Zellen in Blastozyten erzeugt (Jänisch, R.: Science 240, 1468 (1988); Palmiter, R.D. und Brinster, Ann. Rev. Genet. 20, 465 (1986); R.L. Brinster und R.D. Palmiter, Harvey Lectures, Series 80 (Liss, New York, 1986), pp 1-38).

Inzwischen ist die Einführung von fremder genetischer Information in die Keimbahnen für eine Reihe von Tierarten beschrieben worden. So etwa z.B. bei Hühnern (Bosselman et al., Science 243, 533 (1989)), Fischen (Brem, G., Brenig, B., Hoerstgen-Schwark, G., und Winnacker, E.L.: Aquaculture 68, 209 (1988)), Kaninchen (R.E. Hammer et al., Nature 315, 680 (1985); G. Brem et al., Zuchthygiene 20, 251 (1985)), Schafen (A.J. Clark et al., Bio/Technology 7, 487 (1989); J. Simons et al., Bio/Technology 6, 179 (1988); C.E. Rexroad, Jr. et al., Mol. Reprod. Dev. 1, 164 (1989)) und Schweinen (G. Brem et al., Zuchthygiene 20, 251 (1985); K.M. Ebert et al., Mol. Endocrinol. 2, 277 (1988); Brem, G., Brenig, B., Müller, M., Kräußlich, H. und Winnacker, E.L.: Occ. Publ. Br. Soc. Anim. Prod. 12 (1988) 15-31).

In Kaninchen wurde das Kaninchen c-myc Gen unter Kontrolle von regulatorischen Sequenzen eines Immunglobulin-Gens exprimiert, was die Induktion von Leukämie in transgenen Kaninchen zur Folge hatte (Knight, K.L., Spicker-Polat, H., Kazdin, D., Oi, V.T.: Proc. Natl. Acad. Sci. USA 85 (1988) 3130-3134).

Es wurden auch transgene Kaninchen erzeugt, welche das menschliche Somatotropin Gen exprimieren (Enikolopov et al., Dokl. Acad. Nauk SSSR, 299 (1988) 1246-1249). Ebenso wurde Expression des "human growth hormone releasing factor" in transgenen Kaninchen beschrieben (Gataryan et al., Dokl. Acad. Nauk SSSR 305 (1989) 726-728).

Es wurde die Expression von Rinder-Wachstumshormon in transgenen Schweinen gezeigt und der Effekt auf das Wachstum der transgenen Tiere untersucht (J. Anim. Sci. 66 (Suppl 1) 267 (1988)).

Es wurden auch transgene Schweine erzeugt, welche das Oberflächen Antigen des menschlichen Hepatitis B Virus exprimieren. (Dokl. Akad. Nauk SSSR 306 (1989) 206-209).

Gene für die leichte und die schwere Kette von Antikörpern mit definierter Spezifität (gerichtet gegen Nitrophenol, Trinitrophenol, Phosphorylcholin) wurden aus den jeweiligen Hybridom-Linien isoliert und in die Keimbahn von Mäusen eingeführt. Die Gene für die leichte und schwere Kette wurden in den jeweiligen transgenen Mäusen in Form von rekombiniertem Antikörper exprimiert (Rusconi, S. und Köhler, G.: Nature 314, 330 (1985; Grosschedl, R., Weaver, D., Baltimore, D. und Constantini, F.: Cell 38 (1984), 647; U. Storb et al., J. Exp. Med. 164 (1986) 627; Ritchie, K.A., Brinster, R.L. und Storb, U.: Nature 312, 517 (1984); Weaver, D., Constantini, T., Imanishi-Kari, T. und Baltimore, D.: Cell 42, 117 (1985); Iglesias. A., Lamers, M. und Köhler, G.: Nature 330, 482 (1987); Neuberger, M.S., Caskey, H.M., Petersson, M., Williams, T., Surani, M.A.: Nature 338, 350 (1989).

In einigen der obigen Literaturstellen wurden Gene nur für die leichte oder nur für die schwere Kette eines Antikörpers in die Keimbahn von Mäusen eingebracht. Bei diesen Untersuchungen zeigte sich, daß das rearrangierte Transgen das Rearrangement der endogenen Immunglobulin-Gene inhibiert. Es wurde auch nur eine relativ geringe Expression der eingebrachten Antikörper-Gene (bis ca. 5 bis 10 µg/ml Serum) in den transgenen Mäusen gefunden. Bei anderen Tierarten ist die Expression von Immunglobulin-Genen nach Einbringen in die Keimbahn bisher noch nicht beschrieben worden.

Aufgabe der vorliegenden Erfindung war es, ein Verfahren zu entwickeln, bei dem man Antikörper in größerer Ausbeute aus transgenen Tieren gewinnen kann.

Die erfindungsgemäße Aufgabe wird gelöst durch ein Verfahren zur Herstellung einer befruchteten transgenen Eizelle eines Schweins oder Kaninchens, verwendbar zur Gewinnung von Antikörper produzierenden transgenen Schweinen oder Kaninchen, wobei DNA-Sequenzen, die komplette Gene für die leichte und schwere Kette eines Antikörpermoleküls enthalten und frei sind von bakteriellen Fremdsequenzen, in den männlichen Pronukleus einer befruchteten Eizelle eines Schweins oder Kaninchens mittels Mikroinjektion eingebracht werden. Die Nachkommen können gezüchtet werden und aus ihnen kann in üblicher Weise der Antikörper gewonnen werden.

Für die erfindungsgemäße Expression der Antikörper in lymphoiden Zellen werden bevorzugt Immunglobulin-Promotor und Enhancer-Elemente verwendet. Die zu exprimierenden Gene werden auf übliche Weise in einen prokaryontischen Vektor subkloniert und in geeigneten Wirtszellen vermehrt. Die entsprechenden Gene werden nach Spaltung des Vektors mit jeweils geeigneten Restriktions-Nukleasen frei von prokaryotischen Sequenzen isoliert und in befruchtete Eizellen injiziert. Dabei kann man zirkularisierte oder bevorzugt linearisierte DNA verwenden. Auch nach Deletion von Intron-Sequenzen (eines oder mehrerer) auf den zu exprimierenden Genen durch in vitro Mutagenese sollte Expression im transgenen Tier erfolgen. Im Fall der Verwendung von komplementärer DNA (cDNA) zur Expression der leichten und schweren Ketten eines Antikörpers können Immunglobulin-Promotor und Enhancer Sequenzen auf den Expressionsvektoren vor die zu exprimierende cDNA kloniert werden. Auch die Fusion von genomischen Fragmenten mit cDNA ist möglich (Gillies, S.D. et al., Bio/Technology 7 (1989) 799-804; Orlandi et al., Proc. Nat. Acad. of Sci. USA 86 (1989) 3833-3837). Eine weitere Verbesserung der Expression der Antikörper-Gene ist gegebenenfalls auch durch Verwendung von in den nicht-kodierenden Regionen der Antikörper-Gene gelegenen Regulations-Elementen möglich. So wurde für menschliche K Gene (leichte Kette) kürzlich ein in der 3'-untranslatierten Region gelegenes Enhancer-Element beschrieben (Meyer, K.B. und Neuberger, M.S. EMBO J. 8 (1989) 1959-1965, für Maus λ1 Ketten wurde ebenfalls in der 3'-untranslatierten Region ein Enhancer-Element beschrieben (Bich-Thuy, L. und Queen, C., Nucl. Acids Res. 17 (1989) 5307-5321).

Unter den Begriff "Antikörper" fallen:

### a) Vollständige Antikörper verschiedener Spezies:

Die Expression ist durch Einbringen der kompletten Gene für die leichte und schwere Kette des Antikörpers in befruchtete Eizellen des Schweins oder Kaninchens möglich, wobei alle Antikörper-Isotypen prinzipiell exprimiert werden können. Bevorzugt ist die schwere Kette eine γ-Kette und die leichte Kette eine K-Kette.

Die Antikörper können von der Maus, Ratte oder bei Verwendung von Eizellen des Schweins auch aus Kaninchen stammen. Besonders bevorzugt ist jedoch die Expression menschlicher Antikörper.

Menschliche Antikörper haben großes diagnostisches und therapeutisches Potential. Sie sind bei der Verwendung für therapeutische Zwecke Maus-Antikörpern oder gentechnologisch hergestellten chimärisierten oder humanisierten Antikörpern vorzuziehen, da bei ihnen nach therapeutischer Applikation eine Immun-Antwort des Organismus nicht zu erwarten ist. Die Konstruktion von stabilen, gut produzierenden humanen B-Zell-Linien oder Hybridomen ist jedoch in der Praxis mit großen Problemen behaftet.

Für menschliche Antikörper kodierende Gene oder cDNA der leichten und schweren Ketten sind nach üblichen Verfahren aus menschlichen Hybridom-Zellen erhältlich.

Die Gewinnung menschlicher Antikörper und ihr therapeutischer Anwendungsbereich ist in folgenden Literaturstellen beschrieben:

James, H. und Bell, G.T., (1987), Human monoclonal antibody production. Current status and future prospects. J. Immunol. Methods 100, 5-40;

Boyd, J.E. and James, K. (1988), Human monoclonal antibodies: Their potential, problems and prospects. In: Advances in Biotechnological Processes. Vol. II. Mizraki, A. (Ed.) A.R. Liss, Inc., New York;

Larrick, J.W. and Bourla, J.M. (1986), The prospects for the therapeutic use of human monoclonal antibodies. J. Biol. Res. Mod. 5 (1986) 379-393.

### b) Chimärisierte und humanisierte Antikörper:

Unter chimärisierten Antikörpern versteht man hier Moleküle mit variablen Regionen von der Maus oder anderen Tierspezies (inklusive D = Diversity und J = joining Segmenten) und konstanten Regionen einer anderen Spezies, insbesondere des Menschen. Diese Antikörper werden gentechnologisch hergestellt, ebenso wie die humanisierten Antikörper, bei denen in Gene für menschliche leichte und schwere Ketten die hypervariablen CDR-Regionen (complementarity determining regions) z.B. eines entsprechenden Maus- oder Ratten- Antikörpers eingesetzt werden (= Austausch der humanen CDR-Regionen gegen solche des entsprechenden Maus- oder Ratten-Antikörpers). Diese Technik wird als CDR-grafting bezeichnet. CDR-Regionen sind diejenigen Domänen eines Antikörper Moleküls, welche die Affinität zum jeweiligen Antigen determinieren.

Nach dem erfindungsgemäßen Verfahren ist es möglich, im transgenen Schwein oder Kaninchen sämtliche in Frage kommenden Isotypen von chimärisierten Antikörpern zu exprimieren.

Die Gewinnung von chimärisierten Antikörpern durch CDR-grafting ist bei Jones, P.T., Dear, P.H., Foote, J., Neuberger, M.S. and Winter, G., (1986), Replacing the complementary-determining regions in a human antibody with those from a mouse antibody, Nature 321 (1986) 522-525, beschrieben.

Chimärisierte Antikörper haben großes Potential bei der Behandlung maligner Erkrankungen. Dabei erkennen die Antikörper Epitope auf Tumorzell-spezifischen Antigenen (Sun et al., Proc. Natl. Acad. Sci. USA 84 (1987) 214-218; Nishimura, Y. et al., Cancer Res. 47 (1987), 999-1005).

### c) Heterobispezifische Antikörper;

Heterobispezifische Antikörper haben großes therapeutisches Potential, zum Beispiel bei der Elimination von Virus-infizierten Zellen. Ein heterobispezifischer Antikörper erkennt z.B. ein Epitop von auf der Zelloberfläche präsentierten Antigenen und auf zytotoxischen T-Zellen (Gilliland, L.K., Clark, M.R. und Waldmann, H.: Proc. Natl. Acad. Sci. USA 85 (1988) 7719-7723; Staerz, U.D., Yewdell, J.W. und Bevan, M.: Eur. J. Immunol. 17 (1987) 571-574). In der EP-A 0 388 964 wurde ein Verfahren zur gentechnologischen Herstellung von heterobispezifischen Antikörpern beschrieben. Die Expression im transgenen Schwein oder Kaninchen setzt die Isolierung der Gene für die leichten und schweren Ketten beider Antikörper und anschließende Injektion in befruchtete Eizellen voraus. Dabei müssen die beiden Antikörper nicht unbedingt denselben Isotyp (z.B. IgGl) besitzen. Es ist nach dem erfindungsgemäßen Verfahren möglich, heterobispezifische Antikörper mit verschiedenen Isotypen aus transgenen Schweinen oder Kaninchen zu gewinnen und ihre Eigenschaften bezüglich CDC (complement dependent cytotoxicity) und ADCC (antibody-dependent cell-mediated cytotoxicity) zu untersuchen.

### d) Antikörper-Fragmente:

Für die Expression in transgenen Schweinen kommen ebenfalls Teildomänen von Antikörpern in Frage, wie z.B. F(ab')₂, Fab Fragmente, der Fc-Teil
oder Fv Fragmente. Derartige Antikörper-Fragmente können gewonnen werden, wenn anstelle der vollständigen, für Antikörpermoleküle kodierenden DNA-Sequenzen nur die Teilsequenzen in das transgene Schwein oder Kaninchen mikroinjiziert werden, die für das jeweilige Antikörper-Fragment kodieren. Zur Definition der einzelnen Fragmente siehe: Roitt, I., Brostoff, J. und Male, D: Immunology. Gower Medical Publishing, London New York, 1985. F(ab')₂ oder Fab Fragmente sind von Bedeutung für diagnostische Anwendungen, wie zum Beispiel beim Tumor-Imaging.

### e) Fusionsproteine mit Domänen von Antikörper-Molekülen:

Unter diesem Begriff sind Fusionen aus dem Antigenbindenden Teil eines Antikörpers und der signaltransduzierenden Region (Transmembran-Domäne und cytoplasmatische Domäne) von anderen Mitgliedern der Immunglobulin-Superfamilie (z.B. T-Zell-Rezeptor oder Fc-Rezeptoren) zu verstehen. Solche Moleküle könnten als mögliche Biosensoren große praktische Bedeutung erlangen. Auch die Expression von heterologen Fusionsproteinen ist nach dem erfindungsgemäßen Verfahren durch Mikroinjektion der entsprechenden DNA-Sequenzen in eine Eizelle des Schweins oder Kaninchens möglich. Zum Beispiel kann eine Fusion aus der Antigen bindenden Domäne eines Antikörpers mit einer Domäne erfolgen, welche nach Antigen-Bindung eine enzymatische Funktion vermittelt (z.B. Tyrosin-kinase Domäne des Insulin-Rezeptors oder des EGF-Rezeptors oder Guanylat-Cyclase-Domäne des ANF-Rezeptors).

Auch therapeutisch relevante Fusionsproteine, wie Fusionen aus Domänen des CD4-Antigens, dem Rezeptor für das HIV-Virus auf menschlichen T-Lymphozyten und Domänen von Antikörper Molekülen, welche wichtige Effektor-Funktionen vermitteln (z.B. Komplement-Bindung, Zell-Lyse) können nach dem erfindungsgemäßen Verfahren mit hoher Ausbeute in transgenen Schweinen exprimiert werden.

### f) Mutierte Antikörper:

Dazu gehören Antikörper, die durch gentechnologische Manipulationen eine verbesserte Affinität zum Antigen besitzen als der Ausgangs-Antikörper. Dazu gehören auch Antikörper (durch gentechnologische Manipulation) mit verändertem Komplement-Bindungs-Verhalten, verändertem Bindungsverhalten bezüglich Fc-Rezeptoren auf Makrophagen und Monozyten, sowie solche mit veränderten zytolytischen Eigenschaften. Auch diese Gruppe von Antikörper-Molekülen kann nach dem erfindungsgemäßen Verfahren in transgenen Schweinen oder Kaninchen exprimiert werden.

Neben der Gewinnung von Antikörpern aus dem Serum ist es durch das erfindungsgemäße Verfahren auch möglich, eine Immunisierung von transgenen Schweinen oder Kaninchen zu bewirken. Gene für Antikörper gegen bestimmte Antigene, insbesondere Antigene, die für diese Tiere gefährliche Krankheiten hervorrufen, z.B. Influenza oder Schweinepest, können mit dem erfindungsgemäßen Verfahren in die Keimbahn dieser Tiere mit hoher Effizienz eingebracht werden. Auf diese Weise kann man Tiere züchten, die gegen bestimmte Infektionen resistent sind.

Weiterhin ist auch die Sekretion von therapeutisch relevanten Proteinen in die Milch durch das erfindungsgemäße Verfahren möglich.

Antikörper-Moleküle der Klasse IgA können in die Milch sezerniert werden (sekretorisches IgA = sIgA). Durch Fusion mit IgA ist es möglich, auch andere Proteine, z.B. Koagulations-Faktoren wie Faktor VIII oder Faktor IX, oder Enzyme, wie z.B. menschlichen PlasminogenAktivator vom Gewebe-Typ in die Milch von transgenen Tieren zu sezernieren. Durch Einbauen entsprechender Enzym-Spaltstellen zwischen dem sekretorischen Antikörper und dem Fusionspartner (z.B. Kollagenase, Faktor Xa) kann das authentische Molekül nach Isolierung aus der Milch und anschließende enzymatische Spaltung gewonnen werden.

Aus den vorhergehenden Ausführungen wird klar, daß die vorliegende Erfindung auch ein Verfahren zur Herstellung von transgenen Tieren umfaßt, wobei DNA- Sequenzen, die komplette gene für die leichte und schwere kette eines antikörpermoleküβ enthalten und frei sind von bakteriellen Fremdsequenzen, in den männlichen Pronukleus einer befruchteten Eizelle eines Schweins oder Kaninchens mittels Mikvoinjektion eingebracht werden. Die Eizellen können in den Eileiter eines weiblichen Tieres implantiert und die Nachkommen gezüchtet werden. Beispielsweise kann man hierfür DNA-Sequenzen verwenden, die für Antikörper kodieren, deren Antikör peraktivität gegen ein Antigen gerichtet ist, das eine für die betreffende Tierart (Schwein oder Kaninchen) gefährliche Krankheit hervorruft. Derartige Tierkrankheiten sind dem Fachmann bekannt, ebenso die sie verursachenden Antigene. Mit Hilfe dieser bekannten Antigene lassen sich auf an sich übliche Weise Antikörper erzeugen, deren DNA-Sequenz dann auf bekannte Weise (z.B. druch Screening einer Genbank) erhalten werden kann. Anschließend können diese Antikörper kodierenden DNA-Sequenzen zur Herstellung von transgenen Tieren nach dem erfindungsgemäßen Verfahren verwendet werden können. Auf diese Weise lassen sich z.B. transgene Tiere herstellen, die eine Immunität gegen bestimmte Krankheiten besitzen.

Weiterhin ein Gegenstand der vorliegenden Erfindung ist das transgene Tier (d.h. ein Xaninchen oder Schwein), welches durch ein erfindungsgemäßes Verfahren erhältlich ist. Beispiele für transgene Tiere gemäß vorliegender Erfindung sind etwa solche, die den Antikörper A20/44 produzieren, bei dem es sich um einen anti-idiotypischen Antikörper handelt, der in der EP-A 0 388 964 beschrieben ist.

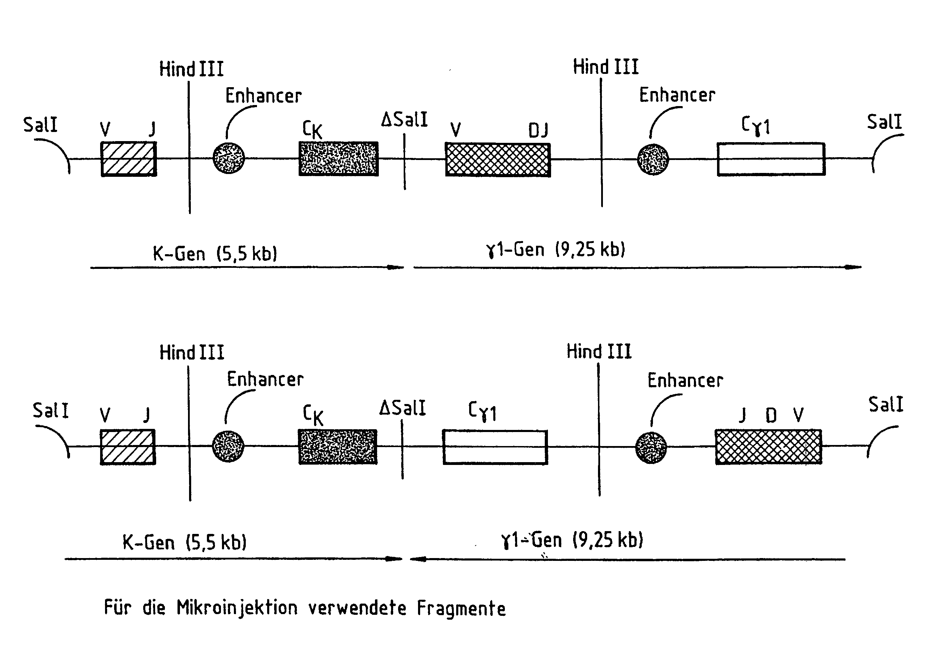

Folgende Beispiele sollen die Erfindung in Verbindung mit Abbildung 1 näher erläutern. Es zeigt:
- Abb. 1: die für die Mikroinjektion verwendeten Antikörpergene.

### Beispiel1

### Herstellung von Antikörper-DNA-Sequenzen

Die Ausgangsvektoren sind die Plasmide pBMS1 (DSM5229) und pBMS2 (DSM5230), welche in der EP-A 0 388 964 (Verfahren zur Herstellung von heterobispezifischen Antikörpern) beschrieben sind. Die Vektoren enthalten das K und γ₁ Gen eines Maus-Antikörpers in unterschiedlichen Orientierungen zueinander. Diese Gene wurden aus einer Hybdridom-Zell-Linie isoliert, welche einen IgGl Antikörper mit der Bezeichnung A20/44 sezerniert (Sablitzky, F., Wildner, G. und Rajewsky, K.: EMBO J. 4 (1985) 345-350; Sablitzky, F., Weisbaum, D. und Rajewsky, K.: EMBO J. 4 (1985) 3435-3437; Kocks, C. and Rajewsky, K.: Proc. Natl. Acad. Sci. USA 85 (1988) 8206-8210). A20/44 ist ein anti-idiotypischer Antikörper, welcher gegen einen für das Hapten NP (4-hydroxy-3-nitro-phenylazetat) spezifischen Antikörper gerichtet ist. Der letztere Antikörper besitzt eine λ-Kette als leichte Kette und eine γ₂ₐ-Kette als schwere Kette (IgG2a).

Die in der EP-A 0 388 964 beschriebenen Vektoren enthalten das K-Gen als 5,5 kb SalI-Fragment, das γ1-Gen als 9,25 kb SalI-Fragment. Die Vektoren enthalten ferner Expressions-Kassetten für die Phosphotransferase neo und Maus-Dihydrofolat-Reduktase unter der Kontrolle des frühen Promotors von SV40.

pBMS1 und pBMS2 (enthalten jeweils 3 SalI-Stellen) wurden mit SalI partiell gespalten, mit Nuklease S1 behandelt (1 µg partiell mit SalI gespaltene DNA wird mit 10 Units S1-Nuclease 60 Minuten bei 30°C in 50 mmol/l Natriumacetat, pH 4,5; 1 mmol/l ZnSO₄ und 0,5 Gew.-% Glycerin inkubiert), das linearisierte Plasmid auf einem niedrig-schmelzenden Agarose-Gel isoliert und die Enden anschließend mit T4- Ligase ligiert. Die Ligierungsansätze wurden in den E.coli Stamm HB101 transfektiert und Ampicillin-resistente (50 µg/ml) Kolonien auf Agarplatten isoliert. Durch Analyse mit Restriktions-Endonukleasen wurden diejenigen Plasmide ermittelt, bei denen durch oben beschriebene Manipulation die zwischen dem K und dem γ1-Gen gelegene SalI-Stelle deletiert worden war. Die erhaltenen Plasmide werden als pBMS1 (ΔSal) und pBMS2 (ΔSal) bezeichnet.

pBMS1 (ΔSal) und pBMS2 (ΔSal) wurden mit SalI gespalten und das SalI-Fragment, welches das K und γ1-Gen des Antikörpers enthält auf einem niedrig-schmelzenden Agarose-Gel isoliert und anschließend gelöst in 10 mM TRIS·HCl, pH 7,5, 0,25 mM EDTA.

Damit wurden die Antikörper-Gene frei von Vektor-Sequenzen isoliert für das anschließende Mikroinjektions-Experiment (siehe Abbildung 1).

### Beispiel 2

### Erstellung transgener Säugetiere

Die Erstellung transgener Säugetiere umfaßt die Herstellung injizierbarer DNA-Lösung, die Gewinnung befruchteter Eizellen und Embryonen, die Mikroinjektion der DNA-Lösung in Vorkerne oder Kerne, den Transfer der injizierten Zygoten auf synchronisierte Empfängertiere und die Untersuchung der geborenen Tiere auf Integration. Dabei sind für die einzelnen Säugerspezies wie Maus, Kaninchen und Schwein einige speziesbedingte Unterschiede bei der Vorbereitung der Spender- und Empfängertiere, der Gewinnung und dem Transfer der Embryonen sowie der Mikroinjektion zu beachten.

### 1. Herstellung einer injizierbaren DNA-Lösung

Nach der DNA-Fragmentisolierung und der Bestimmung des DNA-Gehaltes wird die DNA-Lösung mit Tris-Puffer so verdünnt, daß pro pico Liter Lösung bis zu 1000 Kopien des Genkonstruktes enthalten sind. Alle für die Herstellung der DNA-Mikroinjektionslösung verwendeten Lösungen müssen frei von partikulären Verunreinigungen sein, damit ein Verstopfen der Injektionspipetten vermieden wird.

### 2. Gewinnung der Embryonen

Um die Ausbeute an Embryonen zu erhöhen, werden die Spendertiere in aller Regel superovuliert.

### - Maus

Weiblichen Mäusen, die mindestens 6 Wochen alt sind, werden zur Induktion der Superovulation 5-10 IE PMSG (pregnant mare serum gonadotropin) injiziert. 48 Stunden später erhalten sie 5-10 IE HCG (human choriongonadotropin) und werden mit fertilen Böcken angepaart. Am nächsten Morgen werden die Plaque positiven Mäuse getötet und nach Öffnung der Bauchhöhle werden die Eileiter herausgenommen. Durch Aufreißen der Ampulle mit feinen Pinzetten oder durch Spülen der Eileiter werden die Embryonen gewonnen und in Embryokulturmedium, dem Hyaluronidase zugesetzt wurde, überführt. Die Eizellen werden nach Entfernung der Kumuluszellen gewaschen und bis zur Mikroinjektion kultiviert.

### - Kaninchen

Geschlechtsreife Spenderkaninchen erhalten zur Superovulation eine einmalige Gabe von 10-40 IE PMSG (pregnant mare serum gonadotropin) pro kg Körpergewicht. Dieser Superovulation sollte eine 21-tägige Einzelhaltung oder eine Vorsynchronisation (120 IE HCG oder 0,8 µg-GnRH (Gonadotropin-releasing factor)) vorausgehen. 72 bis 76 Stunden nach der PMSG-Injektion werden die Kaninchen zweimal im Abstand von einer Stunde künstlich besamt oder im Natursprung belegt. Unmittelbar danach erhalten sie zur Ovulationsinduktion 120-180 IE HCG i.v. Die Embryogewinnung erfolgt 19 bis 21 Stunden nach der Belegung durch Schlachttötung der Spenderkaninchen. Die Embryonen werden durch Spülung der Eileiter mit Kulturmedium für Kaninchenembryonen (BMS + 20 % FKS oder PBS + 20 % FKS) vom Fimbrientrichter in Richtung Uterushornende gewonnen. Falls erforderlich, wird der noch vorhandene Cumulus Oophorus durch Hyaluronidasebehandlung entfernt. Die Embryonen werden gewaschen und bis zur Mikroinjektion kultiviert.

### - Schwein

Beim Schwein können auch prepuberale Jungsauen mit einem Gewicht von 60 bis 90 kg für die Embryogewinnung benutzt werden. Am Tag 0 werden die Spendertiere umgestallt und erhalten am Abend eine Applikation von 1250 IE PSMG. 72 Stunden danach wird die Ovulation mit Hilfe von 750 IE HCG eingeleitet. 24 und 36 Stunden nach der HCG-Applikation werden die Tiere besamt. Die Gewinnung der Embryonen erfolgt 24 bis 27 Stunden nach der Besamung. Zur chirurgischen Embryogewinnung werden die Tiere mit 160 mg Azaperon (Stressnil) und 400 mg Metomidathydrochlorid (Hypnodie) narkotisiert. Nach Vorbereitung des Operationsfeldes wird die Haut median in Höhe der letzten zwei Zitzenpaare mit einem ca. 10 cm langen Schnitt geöffnet und Uterus, Oviduct und Ovar werden vorgelagert. Der Uterus wird ca. 5 cm kaudal des uterotubalen Übergangs stumpf perforiert, so daß eine ca. 5 cm Glaskanüle ins Lumen eingeführt und fixiert werden kann. In den Eileiter wird durch den Fimbrientrichter eine 8 cm lange gebogene Augenkanüle, die mit einem ca. 30 cm langen Gummischlauch verbunden ist, eingeführt und fixiert. Der Eileiter wird mit 50 ml PBS-Lösung gespült. Die Spülflüssigkeit wird in einer Petrischale aufgefangen und auf Embryonen abgesucht. Die Embryogewinnung kann auch nach Schlachttötung der Spendertiere erfolgen.

### 3. Mikroinjektion der DNA-Lösung

Für die Mikroinjektion werden ein Inversmikroskop (Zeiss, ICM 405), zwei Leitz-Mikromanipulatoren und ein Injektionsgerät (Eppendorf) verwendet. Der eine Manipulator trägt eine Haltepipette, mit welcher der Embryo durch Unterdruck fixiert werden kann. Auf dem zweiten Mikromanipulator wird die mit DNA-Lösung gefüllte Injektionspipette in einem Nanostepper fixiert und mit dem Injektionsgerät verbunden. Die Spitze der Injektionspipette hat einen Durchmesser von ein bis zwei µm. Zur Mikroinjektion wird die Pipettenspitze durch die Zona Pellucida, die Zellmenbran und die Kernmembran in das Kernlumen vorgeschoben und ca. 1 bis 2 pl DNA-Lösung werden dort abgesetzt. Die Volumenzunahme des Vorkerns signalisiert eine erfolgreiche Mikroinjektion. Z. T. erfolgt auch eine Mikroinjektion der Kerne von Embryonen im Zweizellstadium.

Im Gegensatz zu Maus oder Kaninchen müssen bei den meisten Nutztierspezies (Schwein, Rind) die Eizellen und Embryonen zur Sichtbarmachung der Vorkerne und Kerne vorbehandelt werden (Zentrifugation bei 15000 g für 3 bis 5 Minuten). Die Mikroinjektion erfolgt in einem Mediumtropfen auf einem Deckglas oder in einer sogenannten Injektionskammer. Nach der Mikroinjektion werden die Eizellen oder Embryonen bis zum Transfer kultiviert.

### 4. Transfer der mikroinjizierten Eizellen

### - Maus

Empfängermäuse werden zur Auslösung einer Scheingravidität mit vasektomierten Böcken über Nacht angepaart. Plaque positive Mäuse werden herausgesucht und für den Transfer narkotisiert. Die vorgelagerte Bursa Ovarica wird mit feinen Pinzetten geöffnet und die in einer Transferpipette aufgezogenen Embryonen (10-15 pro Seite) werden in beide Eileiter übertragen. Die Geburt der Jungen erfolgt 20 bis 21 Tage nach dem Transfer.

### - Kaninchen

Empfängerkaninchen werden 21 Tage vor dem voraussichtlichen Termin zur Zyklussynchronisation einzeln aufgestallt und erhalten zur Induktion einer Pseudogravidität 120 IE HCG oder 0,8 µg GnRH i.m. Am Tag vor dem Transfer erhalten die Empfängerkaninchen zur Ovulationsinduktion 120 IE HCG i.v. Zum Transfer werden die Kaninchen mit 0,4 ml Rompun 2 % pro kg/Körpergewicht und 0,8 ml Ketamin 10 % pro kg/Körpergewicht narkotisiert. Nach Vorbereitung des Operationsfeldes und Entleerung der Harnblase durch transabdominalen Druck werden die Kaninchen in Rückenlage angebunden und in Schräglage fixiert. Unmittelbar kaudal des Nabels wird die Bauchhöhle in einer Länge von ca. 4-5 cm geöffnet. Ovar, Eileiter und craniales Uterushorn werden vorgelagert und nach Kontrolle der Ovarreaktion werden die mikroinjizierten Embryonen mit Hilfe einer Transferpipette ca. 2 bis 2,50 cm tief in die Eileiterampulle transferiert. Pro Seite werden zwischen 8 und 15 Embryonen transferiert. Nach Verschluß der Bauchhöhle wird die Operationswunde durch eine Hautfaltendecknaht geschützt. Die Geburt der Jungtiere erfolgt 29 bis 31 Tage nach dem Transfer.

### - Schwein

Auch als Empfängertiere können beim Schwein prepuberale Jungsauen verwendet werden. 12 Stunden nach den Spendertieren erhalten die Empfängertiere 750 IE PMSG und nach weiteren 72 Stunden 750 IE HCG. Diese 12 stündige Asynchronität zwischen Spender- und Empfängertier verbessert die Überlebenschancen der Embryonen nach dem Transfer. Parallel zur Besamung bei den Spendertieren wird bei den Empfängertieren eine Brunstbeobachtung durchgeführt. 135 bis 136 Stunden nach Beginn des Programms (Stunde 0 = PMSG-Injektion bei den Spendertieren) erfolgt der Embryotransfer. Alle injizierten Embryonen (35-45 Stück) werden durch chirurgischen Transfer in einen Eileiter eines Empfängerschweines transferiert. Die Embryonen verteilen sich durch Spacing gleichmäßig auf beide Uterushörner. Ein neues Verfahren erlaubt auch den unblutigen Transfer von Embryonen in den Schweineuterus. Die Geburt der Jungtiere erfolgt 113 bis 116 Tage nach dem Transfer.

### 5. Untersuchung auf Integration

Zur Untersuchung auf Integration der injizierten DNA müssen von den geborenen Tieren Gewebeproben (Schwanzgewebe, Blut oder Biopsie) gewonnen werden. Aus diesen Gewebeproben wird hochmolekulare genomische DNA isoliert. Zum Integrationsnachweis werden Slot Blot, Dot Blot oder Southern-Blot-Analysen durchgeführt.

Positive Tiere (Tiere mit Integration des Genkonstruktes) werden aufgezogen und nach Erreichen der Zuchtreife an nicht transgene Paarungspartner angepaart. Die aus diesen Anpaarungen entstehenden Nachkommen werden darauf untersucht, ob sie das Transgen von ihrem transgenen Elternteil geerbt haben. Durch Verpaarung hemizygot transgener Tiere werden dann homozygot transgene F2-Nachkommen gezüchtet.

Zur Untersuchung der Expression wird von transgenen Tieren nach Blutentnahme Serum gewonnen. Die Blutentnahme erfolgt durch Punktion des retrobulbären Augenplexus (Maus), des Ohrrandes (Kaninchen), oder der Vena jugularis (Schwein) oder aus anderen zugänglichen Venen bzw. bei der Tötung der Tiere.

### Beispiel 3

### Bestimmung des Antikörper-Titers im Serum der transgenen Tiere

Die Bestimmungsmethode des Antikörper-Titers ist in der EP-A 0 388 964 beschrieben. Mikrotiter-Platten wurden mit Antikörper gegen das Hapten NP (4-Hydroxy-3-nitrophenylazetat) beschichtet. Dieser Antikörper ist von Typ IgG 2a (die leichte Kette ist eine λ Kette, die schwere Kette eine γ 2a Kette). Der Beschichtungs-Puffer bestand aus 0.2 mmol/l Carbonat/Bicarbonat, pH 9.4. Nach 2 Stunden wurden die Platten mit einem Nachbeschichtungs-Puffer (50 mmol/l HEPES, 0.15 mol/l NaCl, 1 % Crotein C, pH 7.0) inkubiert. Alle Reaktionen wurden bei Raumtemperatur unter Schütteln durchgeführt. Die Eichkurve wurde mit einer Antikörper A20/44 (IgG 1) enthaltenden Stammlösung (Verdünnungs-Reihe) erstellt. Die Eichproben und die Seren wurden mit Inkubations-Puffer verdünnt (50 mmol/l HEPES, 0.15 mol/l NaCl, 1 % Crotein C, pH 7.0, 0.2 mol/l Di-Natriumtartrat, 0.75 % Polyethylenglycol (PEG), 0.5 % Pluronic®F 68, 0.75 % PEG 40.000, 0.01 % Phenol) und 2 Stunden bei Raumtemperatur inkubiert. Nach Aussaugen der Vertiefungen und zweimaligem Waschen mit Inkubations-Puffer erfolgte eine einstündige Inkubation mit Konjugat (Konjugat aus Fab-Fragmenten des Antikörpers, welcher gegen NP gerichtet ist und Peroxidase (POD)). Dazu wurde das Konjugat im Inkubations-Puffer auf 150 mU/ml POD-Aktivität verdünnt. Nach Aussaugen und dreimaligem Waschen der Vertiefungen mit Wasch-Puffer (50 mmol/l HEPES, 0.15 mmol/l NaCl, 0.1 % Pluronic®F 68, pH 7.0) wurde 60 Minuten mit ABTS® (2,2'Azino-di-[3-ethyl-benzthiazolin-sulfonat (6) ] als Substrat umgesetzt. Die Extinktion wurde im Photometer (ELISA-Reader) bei 405 nm gegen 490 nm gemessen. Die Konzentration der Proben wurde über die Standard-Eichkurve bestimmt.

**Tabelle**

| Antikörper-Titer im Serum transgener Tiere | | | | | |
|---|---|---|---|---|---|
| Maus Nr. | µg/ml | Kaninchen Nr. | µg/ml | Schwein Nr. | µg/ml |
| 970-28 | 3 | 2644 | 200 | 5814 | 1000 |
| 970-29 | 7 | | | Kontrolle | 0 |
| 970-31 | 18 | Kontrolle | 0 | | |
| 970-32 | 10 | | | | |
| 970-33 | 18 | | | | |
| 970-54 | 30 | | | | |
| 970-55 | 3 | | | | |
| 970-56 | 18 | | | | |
| 970-59 | 3 | | | | |
| 974-1 | 27 | | | | |
| 974-2 | 29 | | | | |
| 974-3 | 18 | | | | |
| 974-4 | 4 | | | | |
| 974-8 | 18 | | | | |
| 974-9 | 100 | | | | |
| 974-10 | 45 | | | | |
| 974-11 | 20 | | | | |
| 974-12 | 120 | | | | |
| 974-13 | 60 | | | | |
| 974-14 | 15 | | | | |
| Kontrolle | 0 | | | | |

Die untersuchten Mäuse sind Nachkommen der beiden seropositiven Mäuse 970 und 974. Eine Reihe von Nachkommen war seronegativ bezüglich der eingeführten Antikörper-Spezifität. Diese sind in Tabelle I nicht aufgeführt. Nach Mikroinjektion wurden alle 39 Kaninchen auf Expression untersucht. Ein Tier exprimierte die gewünschte Antikörper-Spezifität im Serum. Es wurden zwei transgene Schweine (1 Totgeburt) erhalten. Das lebend Geborene exprimierte den gewünschten Antikörper überraschenderweise in sehr hoher Konzentration im Serum (1000 µg/ml).

### Beispiel 4

### Charakterisierung des exprimierten Antikörpers im Schweine-Serum

### a) Isoelektrische Fokussierung und Immuno-Fixation

IEF wurde auf einem Phast-Gel-System (Pharmacia) nach den Anweisungen des Herstellers durchgeführt. Die Seren des transgenen und des Kontroll-Schweins wurden 1:1000 verdünnt und unter nicht-denaturierenden Bedingungen auf das Gel aufgetragen. Der pH-Gradient auf dem Gel (pH 5-8) wurde durch Eichproteine (Pharmacia) visualisiert. Die Laufzeit des Gels betrug 30 Minuten. Anschließend wurde für 45 Minuten mit 100 µg eines polyklonalen Antikörpers (Schaf Anti-Maus Fcγ) in einem Volumen von 150 µl inkubiert und mit einem Zellulose-Acetat Streifen abgedeckt. Die nicht reagierenden Proteine wurden durch Waschen mit 50 mmol/l Kaliumphosphat-Puffer, 0,15 mol/l NaCl, 0,05 % Tween®, pH 7.2 entfernt durch Schütteln über Nacht. Anschließend wurden die Immuno-Komplexe durch Silber-Färbung (Pharmacia-Kit) visualisiert. Als Kontrolle war der aus Ascites aufgereinigte Antikörper A20/44 aufgetragen worden.

Es zeigte sich, daß im Serum des transgenen Schweins dieselben charakteristischen Banden identifiziert werden konnten wie mit dem aufgereinigten Antikörper A20/44. Im Serum des Kontroll-Tieres waren diese charakteristischen Banden nicht nachweisbar.

### b) Aufreinigung von Antikörper A20/44 aus dem Serum des transgenen Schweines

A20/44 ist ein anti-idiotypischer Antikörper gegen einen Antikörper (IgG2a), welcher gegen das Hapten NP gerichtet ist. Letzterer wurde aus Ascites aufgereinigt und an Bromcyan aktivierte Sepharose (Pharmacia) nach Vorschrift des Herstellers gekoppelt. Antikörper A20/44 wurde an einer so hergestellten Affinitäts-Säule nach Auftragen von 100 ml Serum des transgenen Schweines immunadsorbiert, eluiert und anschließend protein-chemisch charakterisiert. Er stimmte in seinen Eigenschaften mit dem aus Ascites-Flüssigkeit aufgereinigten Antikörper A20/44 überein.

## Patentansprüche

1. Verfahren zur Herstellung einer befruchteten transgenen Eizelle eines Schweins oder Kaninchens, verwendbar zur Gewinnung von Antikörper produzierenden transgenen Schweinen oder Kaninchen, **dadurch gekennzeichnet, daß** DNA-Sequenzen, die komplette Gene für die leichte und schwere Kette eines Antikörpermoleküls enthalten und frei sind von bakteriellen Fremdsequenzen, in den männlichen Pronukleus einer befruchteten Eizelle eines Schweins oder Kaninchens mittels Mikroinjektion eingebracht werden.

2. Verfahren nach Anspruch 1, **dadurch gekennzeichnet, daß** die DNA-Sequenzen einen Immunglobulin-Promotor und Enhancer-Sequenzen aufweisen.

3. Verfahren nach Anspruch 1 oder 2, **dadurch gekennzeichnet, daß** die bei der Mikroinjektion eingesetzten DNA-Sequenzen in linearer Form sind.

4. Verfahren nach einem der Ansprüche 1 bis 3, **dadurch gekennzeichnet, daß** der Antikörper ein vollständiger, nativer Antikörper ist.

5. Verfahren nach Anspruch 4, **dadurch gekennzeichnet, daß** der Antikörper eine γ-schwere Kette und eine K-leichte Kette besitzt.

6. Verfahren nach einem der Ansprüche 1 bis 3, **dadurch gekennzeichnet, daß** der Antikörper ein chimärisierter Antikörper ist.

7. Verfahren nach einem der Ansprüche 1 bis 2, **dadurch gekennzeichnet, daß** der Antikörper ein heterobispezifischer Antikörper ist.

8. Verfahren nach einem der Ansprüche 1 bis 3, **dadurch gekennzeichnet, daß** der Antikörper mit einem weiteren Polypeptid fusioniert ist.

9. Verfahren nach einem der Ansprüche 1 bis 3, **dadurch gekennzeichnet, daß** der Antikörper ein mutierter Antikörper ist.

10. Transgenes Tier, **dadurch gekennzeichnet, daß** es ein Schwein oder Kaninchen ist, in dessen Genom heterologe DNA-Sequenzen integriert sind, die komplette Gene für die leichte und die schwere Kette eines Antikörpers enthalten und frei von bakteriellen Fremdsequenzen sind.

11. Transgenes Tier nach Anspruch 10, **dadurch gekennzeichnet, daß** es eine Immunität gegen Krankheiten aufweist.

12. Transgenes Tier nach Anspruch 10, **dadurch gekennzeichnet, daß** es den Antikörper A20/44 produziert.

## Claims

1. Process for the production of a fertilzed transgenic egg of a pig or rabbit, usable for producing antibody producing transgenic pigs or rabbits, **characterized in that** DNA-sequences, containing complete genes of the light and heavy chains of an antibody molecule, and being free of exogeneous bacterial sequences, are introduced by microinjection into the male pronucleus of a fertilized egg of a pig and a rabbit.

2. The process according to claim 1, **characterized in that** the DNA-sequences contain an immunoglobulin promotor and enhancer sequences.

3. The process according to any of claims 1 or 2, **characterized in that** the DNA-sequences used for mircoinjection are in a linear form.

4. The process according to any of claims 1 to 3, **characterized in that** the antibody is a complete, native antibody.

5. The process according to claim 4, **characterized in that** the antibody has a heavy γ-chain and a light κ-chain.

6. The process according to any of claims 1 to 3, **characterized in that** the antibody is rendered a chimeric antibody.

7. The process according to any of claims 1 to 2, **characterized in that** the antibody is a hetero-bispecific antibody.

8. The process according to any of claims 1 to 3, **characterized in that** the antibody is fused to another polypeptide.

9. The process according to any of claims 1 to 3, **characterized in that** the antibody is a mutated antibody.

10. Transgenic animal, **characterized in that** being a pig or rabbit in which genome heterologuous DNA-sequences are integrated containing complete genes for the light and heavy chain of an antibody and are being free of exogeneous bacterial sequences.

11. The transgenic animal according to claim 10, **characterized in that** it has an immunity against diseases.

12. The transgenic animal according to claim 10, **characterized in that** it produces the antibody A20/44.

## Revendications

1. Procédé pour la production d'un ovule transgénique fécondé d'un cochon ou d'un lapin, qui est utilisable pour l'obtention de cochons ou de lapins transgéniques produisant des anticorps, **caractérisé en ce que** des séquences d'ADN, qui contiennent des gènes complets pour la chaîne légère et pour la chaîne lourde d'une molécule d'un anticorps et qui sont exemptes de séquences bactériennes étrangères, sont introduites par la voie de la micro injection dans le pro nucléus masculin d'un ovule fécondé d'un cochon ou d'un lapin.

2. Procédé suivant la revendication 1, **caractérisé en ce que** les séquences d'ADN contiennent un promoteur d'immunoglobuline ainsi que des séquences. amplificatrices.

3. Procédé suivant la revendication 1 ou 2, **caractérisé en ce que** les séquences d'ADN utilisées lors de la micro injection se présentent sous forme linéaire.

4. Procédé suivant l'une des revendications 1 à 3, **caractérisé en ce que** l'anticorps est un anticorps complet et natif.

5. Procédé suivant la revendication 4, **caractérisé en ce que** l'anticorps possède une chaîne γ lourde et une chaîne K légère.

6. Procédé suivant l'une des revendications 1 à 3, **caractérisé en ce que** l'anticorps est un anticorps chimérisé.

7. Procédé suivant l'une des revendications 1 à 2, **caractérisé en ce que** l'anticorps est un anticorps hétérobispécifique.

8. Procédé suivant l'une des revendications 1 à 3, **caractérisé en ce que** l'anticorps est fusionné avec un autre polypeptide.

9. Procédé suivant l'une des revendications 1 à 3, **caractérisé en ce que** l'anticorps est un anticorps ayant subi une mutation.

10. Animal transgénique **caractérisé en ce qu'**il est un cochon ou un lapin dans le génome duquel on a intégré des séquences d'ADN hétérologues qui contiennent des gènes complets pour la chaîne légère et pour la chaîne lourde d'un anticorps et qui sont exemptes de séquences bactériennes étrangères.

11. Animal transgénique suivant la revendication 10, **caractérisé en ce qu'**il présente une immunité contre des maladies.

12. Animal transgénique suivant la revendication 10, **caractérisé en ce qu'**il produit l'anticorps A20/44.
